# EUROPEAN PATENT SPECIFICATION

(11) **EP 0 182 874 B1**
(45) Date of publication and mention of the grant of the patent: **20.10.1993**
(21) Application number: 85902859.9
(22) Date of filing: 20.05.1985
(51) Int. Cl.: G01N 33/50, G01N 33/569

(54) **A REAGENT SYSTEM AND METHOD FOR IDENTIFICATION, ENUMERATION AND EXAMINATION OF CLASSES AND SUBCLASSES OF BLOOD LEUKOCYTES**
REAGENSSYSTEM UND VERFAHREN ZUM NACHWEIS, AUFZÄHLEN UND PRÜFEN DER KLASSEN UND UNTERKLASSEN VON BLUTLEUKOZYTEN
SYSTEME REACTIF ET PROCEDE POUR L'IDENTIFICATION, L'ENUMERATION ET L'EXAMEN DES CLASSES ET DES SOUS-CLASSES DE LEUCOCYTES SANGUINS

(30) Priority: 31.05.1984 US 615961
(43) Date of publication of application: 04.06.1986
(73) Proprietor: COULTER CORPORATION, Hialeah, FL 33010 (US)
(72) Inventor: LEIF, Robert, Cary, Coral Gables, FL 33146 (US); LEDIS, Stephen, Lawrence, Hialeah, FL 33013 (US); FEINBERG, Robert, Irwin, Sunrise, FL 33322 (US)
(74) Representative: Nettleton, John Victor
(86) International application number: US8500954
(87) International publication number: WO8505640

(56) References cited:
- US-A- 3 446 751
- US-A- 3 874 852
- US-A- 3 883 247
- US-A- 3 916 205
- US-A- 4 099 917
- US-A- 4 102 810
- US-A- 4 185 964
- US-A- 4 286 963
- US-A- 4 299 917
- US-A- 4 336 029
- US-A- 4 420 558
- US-A- 4 492 752
- US-A- 4 499 052
- US-A- 4 529 705
- IMMUNOLOGY, vol. 77, no. 8, August 1980, pages 4914-4917; R.A. HOFFMAN et al.: "Simple and rapid measurement of human T lymphocytes and their subclasses in peripheral blood"
- THE JOURNAL OF HISTOCHEMISTRY AND CYTOCHEMISTRY, vol. 25, no. 7, 1977, pages 827-835, The Histochemical Society Inc., US; R.A. THOMAS et al.: "Combined optical and electronic analysis of cells with the AMAC transducers"
- Annals New York Academy of Sciences, vol. 420, issued 1983, D.Y. Mason et al., "Single and Double Immunoenzymatic Techniques for Labeling Tissue Sections with Monoclonal Antibodies", pp. 127-133
- J. Histochem. Cytochem., vol. 25, no. 7, issued 1977, R.A. Thomas et al., "Combined Optical and Electronic Analysis of Cells with the AMAC Transducers", pp. 827-835
- Laboratory Management, issued May 1984, J.R. Downing et al., "Flow Cytometry: Applicants in the Clinical Laboratory", pp. 29-37
- Clin. Chem., vol. 23, no. 8, issued 1977, R.C. Leif et al., "Development of Instrumention and Fluorochromes for Automated Multiparameter Analysis of Cells", pp. 1492-1498
- Acta Cytologya, vol. 19, no. 4, issued 1975, G.C. Salzman et al., "Cell Classification by Laser Light Scattering: ...", pp. 372-377

## Description

This invention relates to immunohematology procedures, and more particularly to a reagent system and a method for the identification of classes as well as subclasses within a class and enumeration of cells within those subclasses of blood leukocytes from a whole blood sample which has been incubated with a fluorescent responsive antibody to a select antigenic determinant on the surface of specified subclasses of blood leukocytes.

It is known that the lymphocyte population of blood leukocytes is subdivided into a number of subclasses which play distinct roles in the immune response. In disease states the relative number of lymphocytes found in various subclasses is likely to change. Hence, the enumeration and identification of the cells in the various subclasses will provide useful information in the study and treatment of disease as described by James R. Downing et al in Laboratory Management, May 1984, pages 29-37.

It is known that several particular subclasses of functionally distinct lymphocytes and other blood leukocytes can be identified on the basis of antigenic determinants on the cell.

Monoclonal antibody techniques have been utilized to produce large quantities of highly purified antibody to various lymphocyte and other leukocyte subclasses. Utilizing such antibodies, it has proved feasible to assay the lymphocytes of an individual to determine the relative number of cells in various subclasses. Further, utilizing direct or indirect techniques, the antibodies can be labeled fluorescently, thereby rendering the samples under consideration amenable to flow cytometric analysis and morphology. More recently, additional monoclonal antibodies have been developed which include several that react with monocytes and granulocytes.

Hansen et al describe in U. S. Pat. 4,284,412, 1981, and in Immunology, Vol. 77, No. 8, pp 4914-4917 (1980) a method and apparatus for automated identification and enumeration of specified lymphocyte subclasses. An anticoagulated whole blood sample or buffy coat sample is incubated with an antibody to a specific lymphocyte subclass of interest. The binding of this antibody is detectable if either it has been coupled with a fluorescent chemical moiety (the direct technique), or if it in turn is specifically bound by another macromolecule to which has been coupled a fluorescent dye moiety (the indirect technique). These fluorescent moieties possess the characteristic of emitting fluorescent light upon illumination with incident laser light. The sample then is lysed using ammonium chloride as the lysing agent. and centrifuged, obtaining a pellet containing leukocytes, residual platelets, and erythrocyte ghosts. The pellet is washed and treated with a fluorescent-labeled antibody (indirect technique). A diluted sample then is subjected to flow cytofluorometric analysis. Four clusters of cells are distinguished. However, only three clusters are found to be due to leukocytes. These clusters were identified as (1) lymphocytes, (2) monocytes and (3) granulocytes. The fourth cluster is identified as aggregates or multiples of platelets and red blood cell debris due to incomplete lysing of the red blood cells.

The lysing techniques described in these references now have been improved by the present invention so as to maintain better the morphology of the immunologically labeled specific leukocytes, improve their stability on storage, and render them more suitable for cytofluorescent analysis, or for other operations such as microscopic examination of stained cells on a slide.

Employed herein are the trademarks COULTER, COULTER CLONE, ISOTON, E.A.SY.l, MDADS and EPICS which are owned by Coulter Corporation, Coulter Electronics, Inc., or Coulter Electronics, Ltd.

The present invention provides a process for identifying classes and preferably also selected subclasses of leukocytes in whole blood, the process comprising:
labeling leukocytes by means of labeled antibodies specific to antigenic determinants on the cell surface; and
treating the sample with a lysing reagent
**characterized by:**
treating the sample with a lysing reagent containing saponin for a predetermined time and under specified temperature conditions such that the erythrocytes are lysed; and
treating the lysed sample with a cross-linking fixing reagent for a predetermined time and under specified temperature conditions such that the physical properties and preferably also the morphological properties of the leukocytes are maintained; and
identifying the classes and preferably also selected subclasses of the leukocytes using flow cytometry and/or microscopic morphology.

By way of example, illustrative embodiments of the invention now will be described with reference to the accompanying drawings by which:

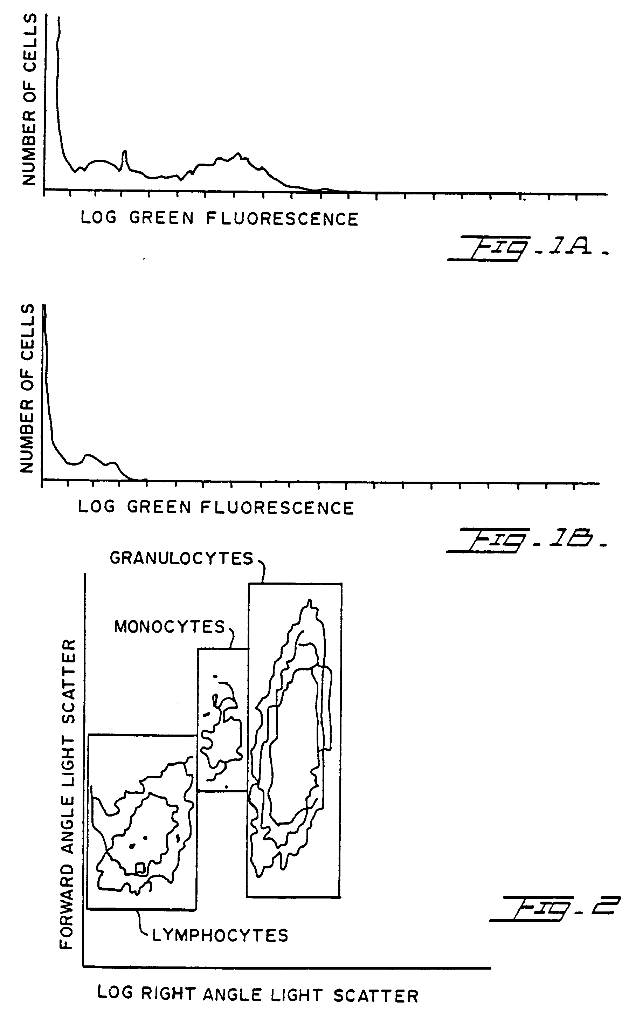

Figs. 1A and 1B show the cell-membrane-antigen histograms produced by flow cytometric analysis. The abscissa represents a value directly proportional to the log of the green fluorescence intensity. The ordinate represents the number of cells counted in each channel on a relative scale.
Fig. 1A shows a sample with T4-FITC, as in Example 1.
Fig. 1B shows a control with no fluorescent antibody added.
Fig. 2 is a contour map in which the abscissa represents the log of the right angle light scatter intensity, and the ordinate represents the forward angle light scatter intensity. The rectangles designate the specific areas of this two dimentional distribution which contain the specific leukocyte cell classes: lymphocytes, monocytes, granulocytes. This contour map is determined from the two dimensional data by a flow cytometer. Each contour line designates the locations on the map where there is essentially the same number of cells, irrespective of light scatter intensity.

This invention relates to an improved reagent system and method for identifying classes and enumerating the cells in selected subclasses within those classes of leukocytes in whole blood, based on utilization of antigenic determinants on the cell surface and their reactivity with labeled antibodies. This invention employs somewhat the general overall procedure described by Hansen et al, but with improvements especially over the conventional lysing and cell fixing procedure.

According to the present invention, the reagent system comprises aqueous solutions of (A) a lysing reagent containing saponin, and (B) a fixing reagent containing a cross-linking agent. The improved reagent system lyses the blood erythrocytes, while maintaining the physical and morphological properties of the blood leukocyte classes, as well as labeling specific leukocyte subclasses. The samples can be analyzed either by the principles of flow cytometry, or by microscopic morphology.

Using saponin as the lysing agent, the method permits fixation with a limited amount of a cross-linking fixative. This is accomplished by lysing at room temperature, or preferably at an elevated temperature, such as 42°C, which selectively destabilizes the erythrocyte membrane and accelerates the lysing reaction. The use of a hypotonic buffer, consisting primarily of potassium salts, also favors lysis.

Glyoxal is preferred over glutaraldehyde as the cross-linking agent, even though the latter is a stronger fixative; because glyoxal, after reacting with a leukocyte, imparts minimal, if any, background fluorescence; whereas glutaraldehyde produces a significant amount of fluorescence. Dialdehydes are preferred over monoaldehydes, such as formaldehyde, which have only a weak cross-linking action for this purpose.

The addition of dimethyl sulfoxide or urea to the fixative improves the reaction, and tends to decrease the amount of fixative required to retard the action of the saponin on the leukocytes. In addition, a temperature drop to approximately 4°C (as by use of an ice bath) slows down the reaction. The cells are weakly fixed and can be sufficiently spread on a slide for cytological preparation.

In copending U. S. Application, S.N. 615,966, filed the same day as the present application, a somewhat analogous lysing method is described, but such method does not include immunological techniques for utilizing antigenic determinants on the cell surface and their reactivity with antibodies that label cells which fluoresce under known circumstances.

This invention concerns a method for identifying classes and enumerating cells in subclasses of leukocytes in whole blood, based on the utilization of antigenic determinants on the cell surface and their reactivity with labeled antibodies, followed by utilization of the principles of flow cytometry or microscopic morphology to identify the cells which have been labeled.

Employed herein are the terms "lysis", or forms thereof, and "morphology". Lysis of the erythrocytes means to render the erythrocytes such that they no longer are detectable by physical techniques, or the erythrocyte residue is such that the signals they generate are sufficiently decreased that they will not interfere with those produced by the blood leukocytes. Morphology means the process of microscopic examination of cells and the determination of their classes and subclasses by either human or artificial intelligence techniques.

According to this invention, a whole blood sample is incubated with diluted monoclonal antibody to label certain subclasses of leukocytes. The erythrocytes then are removed by lysis, and the leukocytes are stabilized by use of a cross-linking fixative, prior to the class and subclass identification. It is preferable to lyse the red blood cells before the steps of identifying the class and subclasses of leukocytes, to avoid the danger that coincident passage of two or more erythrocytes or fragments thereof through counting transducer could be mistaken for white blood cells. A preferred procedure is to lyse the red blood cells prior to the identification of the leukocytes by addition of a lysing reagent to the suspension of cells so as to cause the red blood cells to rupture and release their hemoglobin content into the solution.

The problem is to lyse the red blood cells without damage to the antigen-antibody complexes on the leukocytes, while preserving their cell morphology and producing a stable cellular suspension.

The whole blood sample must be treated in a way that lyses the red blood cells, and at the save time the leukocyte blood cells are maintained in a condition which allows measurements to differentiate them and their subclasses. The lysing reagent needs to act quickly, preferably in less than one minute. The cytogram produced should have the cluster of aggregates or multiplets of platelets, and red cell debris separate from the leukocyte clusters.

In a preferred embodiment of the invention, the whole blood sample is incubated with a fluorochromed antibody to label a specific subclass of leukocytes. The erythrocytes are selectively lysed with a lysing reagent containing saponin, and the directly labeled leukocytes are then fixed, using a fixing reagent containing a dialdehyde. The leukocyte cell suspension then is analyzed by the combination of forward angle and right angle light scattering to yield data representing at least three classes. The positively immunofluorescent leukocyte populations are enumerated with a COULTER® EPICS® flow cytometer. The fixative is a cross-linking or bifunctional fixative, preferably a dialdehyde.

Successful results have been obtained with the following COULTER CLONE® antibodies: T4-FITC, T8-FITC, T11, T11-FITC, B1-FITC and Mo2-FITC. Other antibodies useful for this purpose would include OKT1.PAN, OKT4.IND, and also OKM1.M/G, which reacts with monocytes and granulocytes.

As used herein "fluorescent responsive antibody" refers to antibodies which themselves fluoresce, or antibodies which are labeled to fluoresce under specified stimulation.

A technique has been developed to lyse the blood erythrocytes, while maintaining the physical properties and morphology of the blood leukocytes, and immunologically labeling specific leukocyte subclasses. This technique is unique in that it does not involve centrifugation; yet, it does preserve the cells sufficiently so that they can be stored at 4°C for a day and still be usable for flow analysis. Using this technique, it is possible, for example, to divide T lymphocytes into (1) helper lymphocytes, using COULTER CLONE T4-FITC monoclonal antibody, and (2) suppressor lymphocytes, using COULTER CLONE T8-FITC monoclonal antibody.

The cells prepared by the techniques of this invention also are suitable for other cytological preparations, for instance by centrifugal cytology.

The technique of this invention minimizes the concentration of the saponin lysing reagent required, and consequently permits fixation with a limited amount of fixative. Lysing is accomplished at room temperature, or preferably at an elevated temperature such as 42°C, which selectively destabilizes the erythrocyte membrane and accelerates the lysing reaction. The use of a hypotonic buffer, consisting primarily of potassium salts, also favors lysis.

It has been found that the amount of saponin employed under comparable conditions of lysis can be decreased as the temperature is increased. Thus, the residual amount of saponin in preparations that are not centrifuged can be minimized by increasing the temperature of the lysing reagent.

Bifunctional or cross-linking fixing agents such as glyoxal, glutaraldehyde, carbodiimide, succinaldehyde, Mirsky's reagent, and the like, are suitable for use in the fixing reagent. Mirsky's reagent consists primarily of an impure preparation of glyoxal derived from the chemical treatment of saccharides. Glyoxal is especially preferred in the fixing reagent, because it does not fluoresce. This is an advantage when determining the fluorescence of the antibody binding cells later on in the procedure.

The invention is not limited to fluorescence measurements, but absorbance measurement can be performed such as is described in "Defined Immunofluorescence and Related Cytochemical Methods" by D. Y. Mason, Z. Abdulaziz and B. Falini, Annuals of the New York Academy of Science, Vol. 420, pages 127-138 (1983).

The technique of this invention can be employed with the additional physical measurements of DC and AC impedence. The measurements can be utilized in conjunction with the aforementioned light scattering measurements, or in place of them.

The fixed blood cells are stable when stored cold at about 2 to 4°C prior to the light scattering procedure. A laboratory study established that samples were stable for 24 hours post lyse. For example, using COULTER CLONE antibody T8-FITC, the percent positive cells was 20.3 ± 1.0 immediately after sample preparation, 20.3 ± 0.6 after 20 hours post lyse, and 20.0 ± 0.3 at 24 hours post lyse.

Centrifugation and removal of the supernatant fluid between processing steps is beneficial in that it significantly decreases cell debris, and removes excess loosely bound antibody. However, centrifugation has the disadvantage that it virtually precludes obtaining an absolute count of the cells present, and under some conditions can result in selective cell loss. Although centrifugation can be part of a semiautomated system, it introduces considerable complexity into a completely automated system.

One of the major constraints in the development of the present reagent system is that sample preparation for a flow cytometer, such as an EPICS system, unlike that for a standard hematology analyzer, such as the COULTER® Model S series instrument, is asynchronous. The time between the final sample preparation and measurement can range from almost immediately to the next day.

One other problem is the choice of a suitable control. The exquisite specificity of monoclonal antibodies in some cases for example, when fluoresceinated mouse immunoglobulin G (mouse IgG-FITC) is used as a control, leads to an over-estimation of background fluorescence. Either a preincubation and/or a simultaneous incubation with unlabeled antibody blocks the nonspecific binding sites, and this in many cases can eliminate the need for a control.

Samples containing antibody prepared according to this invention are capable of being prepared employing Romanowsky stain. It is also possible in the case of fluorescent studies to stain the cell with two stains, such as dichlorofluorescin and 4,6-diamino-2-phenylindole (DAPI). Other conventional stains include Mithromycin and Acridine Orange.

The examples which follow illustrate certain of the methods and procedures followed in the invention.

### Example 1

### Cell Preparation: The lyse and fixative reagents are prepared at room temperature, approximately 24°C.

1. The 100 ul of phosphate buffered saline (PBS) is added into a 16 x 100 tube followed by 100 ul of whole blood. The tube contents then are gently mixed by swirling.
2. The 5 micrograms of COULTER CLONE monoclonal T11 antibody is added into the same tube and mixed gently. The tube then is kept at room temperature approximately 24°C, for 20 minutes, with occasional shaking.
3. The sample, since this was an indirect fluorescence technique, was washed twice by centrifugation with 4 ml PBS. The sample then was treated with 85 micrograms of fluoresceinated goat antimouse antibody and incubated for 20 minutes, and washed as before with 4 ml of PBS.
4. The cells were suspended in 1 ml of a solution consisting of 3 g NaCl and 1 g NaHCO₃ and water to one liter.
5. The 100 ul of lyse reagent consisting of: 24 g saponin, 4.0 g NaCl, 1 g sorbic acid and water to one liter then is added to the tube containing blood, including the indirectly labeled T lymphocytes and continuously agitated for eight seconds.
6. At the end of the 8 second lysis, 1000 ul of the fixative reagent is added to the lysed sample. This fixative reagent consisted of: 6.0 g of NaCl, 22 g of glutaraldehyde, brought up to one liter volume with water.
7. The sample which is indirectly labeled with monoclonal antibody, lysed and then fixed must be analyzed with the flow cytometer within 15 minutes, because of the development of glutaraldehyde induced autofluorescence. Filtration of the sample, preferable through a 37 micron mesh, is desirable.

Sample Analysis: The samples are analyzed with the COULTER EPICS V single laser, flow cytometer system. The system configuration is set up as herein explained. The laser emits 500 mw of 488 nm radiation. the preferred filter configuration is a 515 interference acting as a blocking filter for green fluorescence, 488 nm dichroic mirror and an NDl filter for orthogonal light scatter, and a NDl filter for forward angle light scatter. The data are analyzed with a computer system, such as in the COULTER MDADS™ or E.A.SY.l®. The three parameters measured are log fluorescence, low angle light scatter and log right angle light scatter. This analysis procedure also is utilized for each of the following Examples.

### Example 2

### Cell Preparation: The lyse and fixative reagents are prepared at 37°C. The reagents can be kept capped in a water bath throughout the procedure.

1. A 100 ul portion of labeling diluent, consisting of 1.0 g of NaN₃, 1.36 g KH₂PO₄, 1.31 g of K₂HPO₄ and 3.73 g of KCl, brought up to one liter volume with water, is added into a 16 x 100 tube, followed by 100 ul of whole blood. The tube contents are then gently mixed by swirling.
2. Ten micrograms of a non-specific, unlabeled mouse antibody is added to block any non-specific binding of the monoclonal antibody.
3. One microgram of COULTER CLONE monoclonal antibody T4-FITC is added into the same tube and mixed gently. The tube is then placed in a water bath maintained at 37°C for five minutes, with occasional shaking.
4. The 100 ul of a lyse reagent consisting of 4 g saponin, 1.75 g of NaCl, 1.36 g of KH₂PO₄, 1.31 g of K₂HPO₄ and 2.24 g of KCl, brought up to one liter volume with water, is then added to the tube containing blood and antibody, and continuously is agitated in the water bath for one minute.
5. At the end of the one minute lysis, 500 ul of a fixative reagent is added to the lysed sample, mixed gently, and kept in the water bath at 37°C for an additional five minutes, mixing occasionally. This fixative reagent consists of 11.7 g of NaCl, 0.43 g of calcium gluconate, 21 g of glyoxal, 220 g of dimethyl sulfoxide and 25 g of Carbowax 1450 brought up to one liter volume with water.

At this point in time the whole blood has been labeled with monoclonal antibody₃ lysed and then fixed. It is now ready for analysis with the EPICS flow cytometer. Filtration of the sample, preferably through a 37 micron mesh, is desirable. The samples are stabilized at approximately 2°C on ice, if need be, for at least 5 minutes prior to sample analysis. Analysis is as stated at the end of Example 1.

In accordance with the above procedure, but substituting for the COULTER CLONE monoclonal T4-FITC of Example 1, any one of the following COULTER CLONE monoclonal antibodies: T8-FITC; T11-FITC; B1-FITC; Mo2-FITC; similar results are obtained. In each instance the leukocyte subclass which is labeled is the leukocyte which is specific for the antibody employed.

### Example 3

### Cell Preparation: The lyse and fixative reagents are prepared at 24°C. The reagents can be kept capped in a water bath throughout the procedure.

1. The 100 ul of a labeling diluent consisting of 1.0 g of NaN₃, in 1 liter ISOTON® Plus diluent is added into a 16 x 100 tube followed by 100 ul of whole blood. The tube contents are then gently mixed by swirling.
2. COULTER CLONE monoclonal antibody T4-FITC is added in the amount of one microgram into the same tube, mixed gently, and then placed in a test tube rack at room temperature (24°C) for five minutes, shaking occasionally.
3. The 1000 ul of lyse reagent, consisting of 0.5 g of saponin, 3.72 g of KCl, 1.36 g of KH₂PO₄, and 1.31 g of K₂HPO₄, brought up to one liter volume with water, is then added to the tube containing blood and antibody, vortexed gently for 30 seconds and placed in a rack at room temperature (24°C) for five minutes, mixing gently each minute.
4. At the end of the five minute lysis, 1000 ul of the fixative reagent consisting of 12.6 g of NaCl, 220 g of dimethyl sulfoxide, 200 ml of Mirsky's reagent and 600 ml of ISOTON Plus diluent, is added to the lysed sample, mixed gently, and kept at room temperature (approximately 24°C) for an additional five minutes, mixing occasionally. Mirsky's reagent is commercially available from Mirsky's National Diagnostics, Somerville, New Jersey.

At this point in time the whole blood has been labeled with monoclonal antibody, lysed and then fixed. It is ready for analysis with the EPICS flow cytometer. Filtration of the sample, preferably through a 37 micron mesh, is desirable.

The samples are stabilized on ice, if need be, for at least 5 minutes prior to sample analysis, and analyzed as stated at the end of Example 1.

### Example 4

### Cell Preparation: The lyse and fixative reagents are prepared at 42°C. The reagents can be kept capped in a water bath throughout the procedure.

1. The 100 ul of a labeling diluent, consisting of 1.0 g of NaN₃ in 1 liter of ISOTON Plus diluent, is added into a 16 x 100 tube followed by 100 ul of whole blood. The tube contents are then gently mixed by swirling.
2. Four micrograms of COULTER CLONE monoclonal antibody T4-FITC are added into the same tube, mixed gently, and then placed in the water bath maintained at 42°C for five minutes, shaking occasionally.
3. The lyse reagent, consisting of 24 g of saponin, 4.0 g of NaCl, 1.0 g sorbic acid and water to make 1 liter is prepared. Ten milliliters of this lyse reagent is then diluted with 1 liter of lyse diluent consisting of 1.31 g K₂HPO₄ and 1.36 g KH₂PO₄ in distilled water. 1000 ul of the mixture is added to the tube containing blood and antibody, and the tube is continuously agitated in the water bath for one minute.
4. At the end of a 30 second lysis, 1000 ul of the fixative reagent consisting of 12.6 g of NaCl, 200 ml of Mirsky's reagent, 220 g of dimethyl sulfoxide, and 600 ml of ISOTON Plus diluent brought up to 1 liter volume with water, is added to the lysed sample, mixed gently, and kept in the water bath at 42°C for an additional five minutes, mixing occasionally.

At this point in time the whole blood has been labeled with monoclonal antibody, lysed and then fixed. It is ready for analysis with the EPICS flow cytometer. Filtration of the sample, preferably through a 37 micron mesh, is desirable.

The samples are stabilized on ice, if need be, for at least 5 minutes prior to sample analysis and analyzed as stated at the end of Example 1.

### Example 5

### Cell Preparation: The lyse and fixative reagents are prepared at 37°C. The reagents can be kept capped in a water bath throughout the procedure.

1. A 100 ul portion of labeling diluent, consisting of 1.0 g of NaN₃, 1.36 g KH₂PO₄, 1.31 g of K₂HPO₄ and 3.73 g of KCl, brought up to one liter volume with water, is added into a 16 x 100 tube, followed by 100 ul of whole blood. The tube contents are then gently mixed by swirling.
2. Ten micrograms of a non-specific, unlabeled mouse antibody is added to block any non-specific binding of the monoclonal antibody.
3. One microgram of COULTER CLONE monoclonal antibody T8-FITC is added into the same tube and mixed gently. The tube is then placed in a water bath maintained at 37°C for five minutes, with occasional shaking.
4. The 100 ul of a lyse reagent consisting of 4 g saponin, 1.75 g of NaCl, 1.36 g of KH₂PO₄, 1.31 g of K₂HPO₄ and 2.24 g of KCl, brought up to one liter volume with water, is then added to the tube containing blood and antibody, and continuously is agitated in the water bath for one minute.
5. At the end of the one minute lysis, 500 ul of a fixative reagent is added to the lysed sample, mixed gently, and kept in the water bath at 37°C for an additional five minutes, mixing occasionally. This fixative reagent consists of 11.7 g of NaCl, 0.43 g of calcium gluconate, 21 g of glyoxal, 220 g of dimethyl sulfoxide and 25 g of Carbowax 1450 brought up to one liter volume with water.

At this point in time the whole blood has been labeled with monoclonal antibody, lysed and then fixed. It now is ready for analysis with the EPICS flow cytometer equipped with the COULTER CVA, employing the principles proposed for the AMAC III, R. C. Leif et al, Clinical Chemistry 23, 1492-8 (1977); and R. A. Thomas et al, J. Histochemistry and Cytochemistry, Vol. 25, No. 77, pp 827-835 (1977). The designator COULTER "CVA" represents "cell volume accessory" and, in quite simple terms, means that the electronic cell analysis equipment utilizing the well known Coulter principle of particle detection has been utilized and integrated into a flow cytometer. Such multiparameter instrument has been demonstrated commercially. Filtration of the sample, preferably through a 37 micron mesh, is essential. The samples are stabilized on ice, if need be, for at least 5 minutes prior to sample analysis. The parameters measured were forward angle light scattering, right angle light scattering, fluorescein immunofluorescence and electronic cell volume.

### Example 6

### Cell Preparation: The lyse and fixative reagents are prepared at 42°C. The reagents are kept capped in a water bath throughout the procedure.

1. One hundred ul of labeling diluent consisting of 1 g of NaN₃, in 1 liter of ISOTON Plus diluent is added into a 16 x 100 tube followed by 100 ul of whole blood. The contents are then gently mixed by swirling.
2. Four micrograms of COULTER CLONE monoclonal antibody T4-FITC are added into the same tube. The tube is mixed gently, and then placed in the water bath maintained at 42°C for five minutes, shaking occasionally.
3. Then 100 ul of lyse reagent, consisting of 0.4 g saponin, 0.05 g of sorbic acid, 1.36 g of KH₂PO₄, 1.31 g of K₂HPO₄, 3.72 g of KCl, and water to make one liter, is added to the tube containing blood and antibody and continuously agitated in the water bath for 30 seconds.
4. At the end of the 30 second lysis, 1000 ul of a fixative reagent consisting of 12.6 g of NaCl, 220 g of dimethyl sulfoxide, 200 ml of Mirsky's reagent, and 600 ml of ISOTON Plus diluent, is added to the lysed sample, mixed gently, and kept in the water bath at 42°C for an additional five minutes, mixing occasionally.

At this point in time the whole blood has been labeled with monoclonal antibody, lysed and then fixed. It is ready for analysis with the EPICS flow cytometer. Filtration of the sample, preferably through a 37 micron mesh, is desirable. The samples are stabilized on ice, if need be, for at least five minutes prior to sample analysis and analyzed as stated in Example 1.

### Example 7

### Cell Preparation: The lyse and fixative reagents are prepared at 37°C. The reagents can be kept capped in a water bath throughout the procedure.

1. The 100 ul of a labeling diluent, consisting of 1.0 g of NaN₃, 1.36 g of KH₂PO₄, 1.31 g of K₂HPO₄ and 3.73 g of KCl, brought up to 1 liter volume with water, is added into a 16 x 100 tube followed by 100 ul of whole blood. The tube contents are then gently mixed by swirling. To this is then added 10 ul of a staining solution consisting of 0.025 g of 4,6-diamino-2-phenylindole (DAPI) in 10 ml of absolute ethanol.
2. Ten micrograms of a non-specific unlabeled mouse antibody is added to block any non-specific binding of the monoclonal antibody. The tube is mixed gently and then placed in the water bath maintained at 37°C for five minutes, shaking occasionally.
3. One microgram of COULTER CLONE monoclonal antibody T8-FITC is added into the same tube. The tube is mixed gently, and then placed in the water bath maintained at 37°C for five minutes, shaking occasionally.
4. To the tube containing the staining solution, blood and antibody, is added 100 ul of lyse reagent. This reagent consists of 4 g of saponin, 1.75 g of NaCl, 1.36 g of KH₂PO₄, 1.31 g of K₂HPO₄, 2.24 g of KCl, and water to make one liter volume. After the reagent is added, the tube is continuously agitated in the water bath for one minute.
5. At the end of the one minute lysis, 500 ul of a fixative reagent consisting of 11.7 g of NaCl, 0.43 g of calcium gluconate, 21 g of glyoxal, 220 g of dimethyl sulfoxide, 25 g of Carbowax 1450, and water to make one liter, is added to the lysed sample, mixed gently, and kept in the water bath at 37°C for an additional five minutes, mixing occasionally.
   At this point in time the whole blood has been labeled with the monoclonal antibody T8-FITC and DAPI, lysed and then fixed. It is ready for preparation by centrifugal cytology. The samples are stabilized on ice, if need be, for at least 5 minutes prior to sample analysis.
6. A standard microscope slide is prepared with a poly-d-lysine solution consisting of 50 mg of poly-d-lysine having a molecular weight of approximately 700,000, by dipping the slide in the solution, and then drying on a slide dryer maintained at 60°C.
7. A pair of Leif Centrifugal Cytology buckets (U.S. Pat. 4,250,830) is assembled and the lysed blood sample is spun for five minutes at 1,500 rpm. The supernatant fluid is removed, and the sample is washed three times with the labeling diluent.
8. The supernatant fluid is removed and the cells in the labeling diluent on the slide are coverslipped. The slide is now ready for microscopic examination with a mercury arc ultraviolet excitation for DAPI, and visable excitation for FITC.

As can be seen from the preceding examples, the relative volumes of the blood sample 50 to 100 ul, the lysing reagent 100 to 1000 ul, and the fixative 500 to 1000 ul can vary. The lysing reagent 0.24 to 4 g/L and fixative concentrations 0.66 g/L to 40 g/L are inversely related to their volumes and must be scaled up as the volume of all previously added reagents is increased. The quantity of saponin 0.24 to 4.0 g/L also is inversely related to both the temperature 24°C to 60°C and the period for lysis 38 seconds to 5 minutes. The quantity of fixative also is related to whether the sample is to be stored for a prolonged period or used immediately for morphology. For the long term storage, maximum fixation is essential, but for the near immediate use, minimal fixation often is preferable.

It is understood that the illustrative embodiments set forth herein constitute examples of the principles of the present invention, but that numerous alternatives will occur to those of ordinary skill in the art, without departure from the scope of this invention.

## Claims

1. A process for identifying classes and preferably also selected subclasses of leukocytes in whole blood, the process comprising:
labeling leukocytes by means of labeled antibodies specific to antigenic determinants on the cell surface; and
treating the sample with a lysing reagent
**characterized by:**
treating the sample with a lysing reagent containing saponin for a predetermined time and under specified temperature conditions such that the erythrocytes are lysed; and
treating the lysed sample with a cross-linking fixing reagent for a predetermined time and under specified temperature conditions such that the physical properties and preferably also the morphological properties of the leukocytes are maintained; and
identifying the classes and preferably also selected subclasses of the leukocytes using flow cytometry and/or microscopic morphology.

2. The process of claim 1 characterized wherein each of said labeled antibodies consists of an antibody linked to a fluorochrome.

3. The process of claim 2 characterized wherein a combination of physical measurements is employed to isolate leukocyte classes and wherein the fluorescent distribution of each class is measured.

4. The process of claim 3 characterized wherein at least one of said physical measurements is light scattering, electronic impedance, a combination of forward angle and right angle light scattering, or a combination of electronic impedance and right angle light scattering.

5. The process of claim 3 characterized wherein the leukocytes are classified by morphology, and subclassified by fluorescent intensity.

6. The process of any one of claims 1 to 5 characterized wherein said fixing reagent contains glutaraldehyde.

7. The process of any one of claims 1 to 5 characterized wherein said fixing reagent contains a non-fluorescing dialdehyde as the active ingredient.

8. The process of any one of claims 1 to 7 characterized wherein said lysing reagent contains saponin in a concentration of about 0.24 g per liter to about 4 g per liter; or about 0.2 g per liter to about 1.14 g per liter in the lysed sample.

9. The process of any one of claims 1 to 8 characterized wherein said lysing temperature is from about 24°C to about 42°C.

10. The process of any one of claims 1 to 9 characterized wherein said lysing time is 8 seconds to about 5 minutes.

11. A reagent system for use on a whole blood sample for identifying classes and preferably also selected subclasses of leukocytes, the reagent system comprising:
labeled antibodies specific to antigenic determinants on the cell surface for labeling leukocytes;
a lysing reagent, for treating the sample for a predetermined time and under specified temperature conditions, containing an amount of saponin sufficient for lysing the erythrocytes, but leaving the leukocytes substantially intact; and
a fixing reagent, for treating the lysed sample for a predetermined time and under specified temperature conditions, containing a cross-linking agent in such amount that the physical properties and preferably also the morphological properties of the leukocytes are maintained for identifying the classes and preferably also selected subclasses of leukocytes by flow cytometry and/or microscopic morphology.

12. The reagent system of claim 11 characterized wherein said fixing reagent contains glutaraldehyde.

13. The reagent system of claim 11 characterized wherein said fixing reagent contains a non-fluorescing dialdehyde as the active ingredient.

14. The reagent system of any one of claims 11 to 13 characterized wherein said lysing reagent contains saponin in a concentration of about 0.24 g per liter to about 4 g per liter; or about 0.2 g per liter to about 1.14 g per liter in the lysed sample.

15. The reagent system of any one of claims 11 to 14 characterized by antibodies linked to a fluorochrome for labeling the leukocytes.

16. Use of a reagent system comprising:
(i) a lysing reagent containing saponin for lysing erythrocytes in a sample of whole blood while leaving leukocytes substantially intact, classes and preferably also subclasses of leukocytes having been labeled by means of specific antibodies, and
(ii) a fixing reagent containing a cross-linking agent for maintaining the physical properties and preferably also the morphological properties of the leukocytes in the sample,
in the preparation of a sample for identification of classes and preferably also subclasses of leukocytes using flow cytometry and/or microscopic morphology.

17. Use as claimed in claim 16, wherein said fixing reagent contains glutaraldehyde.

18. Use as claimed in claim 16, wherein said fixing reagent contains a non-fluorescing dialdehyde as the active ingredient.

19. Use as claimed in any one of claims 16 to 18, wherein at least one dye is used to stain the leukocytes.

20. Use as claimed in any one of claims 16 to 19, wherein said lysing reagent contains saponin in a concentration of about 0.24 g per liter to about 4 g per liter; or about 0.2 g per liter to about 1.14 g per liter in the lysed sample.

21. Use as claimed in any one of claims 16 to 20, wherein antibodies linked to a fluorochrome are used for labeling the leukocytes.

## Patentansprüche

1. Verfahren zur Identifizierung von Klassen und vorzugsweise auch von ausgewählten Subklassen von Leukozyten im Gesamtblut, umfassend:
Markierung von Leukozyten mittels markierter, für Antigen-Determinanten auf der Zelloberfläche spezifischer Antikörper, und
Behandlung der Probe mit einem Lysierungsreagens,
gekennzeichnet durch
Behandlung der Probe mit einem Saponin enthaltenden Lysierungsreagens für eine vorbestimmte Zeit und unter angegebenen Temperaturbedingungen derart, daß die Erythrozyten lysiert werden, und
Behandlung der lysierten Probe mit einem vernetzenden Fixierungsreagens für eine vorbestimmte Zeit und unter angegebenen Temperaturbedingungen derart, daß die physikalischen Eigenschaften und vorzugsweise auch die morphologischen Eigenschaften der Leukozyten beibehalten werden, und
Identifizierung der Klassen und vorzugsweise auch der ausgewählten Subklassen der Leukozyten unter Verwendung von Flußzytometrie und/oder Mikroskop-Morphologie.

2. Verfahren nach Anspruch 1, worin jeder der markierten Antikörper aus einem an ein Fluorochrom gebundenen Antikörper besteht.

3. Verfahren nach Anspruch 2, worin eine Kombination von physikalischen Bestimmungen zur Isolierung von Leukozytenklassen durchgeführt wird, und worin die Fluoreszenzverteilung jeder Klasse bestimmt wird.

4. Verfahren nach Anspruch 3, worin mindestens eine der physikalischen Bestimmungen eine Bestimmung von Lichtstreuung, elektronischer Impedanz, einer Kombination aus Vorwärtswinkel- und Rechtwinkel-Lichtstreuung oder einer Kombination aus elektronischer Impedanz und Rechtwinkel-Lichtstreuung ist.

5. Verfahren nach Anspruch 3, worin die Leukozyten durch Morphologie klassifiziert und durch Fluoreszenzintensität subklassifiziert werden.

6. Verfahren nach einem der Ansprüche 1 - 5, worin das Fixierungsreagens Glutaraldehyd enthält.

7. Verfahren nach einem der Ansprüche 1 - 5, worin das Fixierungsreagens einen nicht-fluoreszierenden Dialdehyd als den Wirkstoff enthält.

8. Verfahren nach einem der Ansprüche 1 - 7, worin das Lysierungsreagens Saponin in einer Konzentration von etwa 0,24 g pro Liter bis etwa 4 g pro Liter, oder von etwa 0,2 g pro Liter bis etwa 1,14 g pro Liter in der lysierten Probe enthält.

9. Verfahren nach einem der Ansprüche 1 - 8, worin die Lysierungstemperatur etwa 24°C bis etwa 42°C beträgt.

10. Verfahren nach einem der Ansprüche 1 - 9, worin die Lysierungszeit 8 sec bis etwa 5 min ist.

11. Reagenssystem zur Verwendung bei einer Gesamtblutprobe zur Identifizierung von Klassen und vorzugsweise auch von ausgewählten Subklassen von Leukozyten, umfassend:
markierte, für Antigen-Determinanten auf der Zelloberfläche spezifische Antikörper zur Markierung von Leukozyten,
ein Lysierungsreagens zur Behandlung der Probe für eine vorbestimmte Zeit und unter angegebenen Temperaturbedingungen, enthaltend eine Menge von Saponin, die zur Lysierung der Erythrozyten ausreichend ist, die Leukozyten im wesentlichen jedoch intakt läßt, und
ein Fixierungsreagens zur Behandlung der lysierten Probe für eine vorbestimmte Zeit und unter angegebenen Temperaturbedingungen, enthaltend ein Vernetzungsreagens in solcher Menge, daß die physikalischen Eigenschaften und vorzugsweise auch die morphologischen Eigenschaften der Leukozyten beibehalten werden, um die Klassen und vorzugsweise auch die ausgewählten Subklassen von Leukozyten durch Zytometrie und/oder Mikroskop-Morphologie zu identifizieren.

12. Reagenssystem nach Anspruch 11, worin das Fixierungsreagens Glutaraldehyd enthält.

13. Reagenssystem nach Anspruch 11, worin das Fixierungsreagens einen nicht-fluoreszierenden Dialdehyd als den Wirkstoff enthält.

14. Reagenssystem nach einem der Ansprüche 11 - 13, worin das Lysierungsreagens Saponin in einer Konzentration von etwa 0,24 g pro Liter bis etwa 4 g pro Liter, oder von etwa 0,2 g pro Liter bis etwa 1,14 g pro Liter in der lysierten Probe enthält.

15. Reagenssystem nach einem der Ansprüche 11 - 14, worin Antikörper mit einem Fluorochrom zur Markierung der Leukozyten verbunden sind.

16. Verwendung eines Reagenssystems, umfassend:
(I) ein Lysierungsreagens, enthaltend Saponin zur Lysierung von Erythrozyten in einer Gesamtblutprobe, während Leukozyten im wesentlichen intakt bleiben, wobei Klassen und vorzugsweise auch Subklassen von Leukozyten mittels spezifischer Antikörper markiert worden sind, und
(II) ein Fixierungsreagens, enthaltend ein Vernetzungsreagens zur Beibehaltung der physikalischen Eigenschaften und vorzugsweise auch der morphologischen Eigenschaften der Leukozyten in der Probe,
in der Herstellung einer Probe zur Identifizierung von Klassen und vorzugsweise auch von Subklassen von Leukozyten durch Flußzytometrie und/oder Mikroskop-Morphologie.

17. Verwendung nach Anspruch 16, worin das Fixierungsreagens Glutaraldehyd enthält.

18. Verwendung nach Anspruch 16, worin das Fixierungsreagens einen nicht-fluoreszierenden Dialdehyd als den Wirkstoff enthält.

19. Verwendung nach einem der Ansprüche 16 - 18, worin mindestens ein Farbstoff zur Anfärbung der Leukozyten verwendet wird.

20. Verwendung nach einem der Ansprüche 16 - 19, worin das Lysierungsreagens Saponin in einer Konzentration von etwa 0,24 g pro Liter bis etwa 4 g pro Liter, oder von etwa 0,2 g pro Liter bis etwa 1,14 g pro Liter in der lysierten Probe enthält.

21. Verwendung nach einem der Ansprüche 16 - 20, worin an ein Fluorochrom gebundene Antikörper zur Markierung der Leukozyten verwendet werden.

## Revendications

1. Procédé pour identifier des classes et, de préférence, également des sous-classes sélectionnées de leucocytes dans du sang entier, le procédé consistant à :
marquer les leucocytes au moyen d'anticorps marqués spécifiques des déterminants antigéniques à la surface des cellules : et
traiter l'échantillon avec un réactif de lyse caractérisé par :
le traitement de l'échantillon avec un réactif de lyse contenant de la saponine pendant un temps prédéterminé et dans des conditions spécifiées de température, de manière que les érythrocytes soient lysés ; et
le traitement de l'échantillon lysé avec un réactif fixant réticulant pendant un temps prédéterminé et dans des conditions spécifiées de température, de manière que les propriétés physiques et, de préférence, également les propriétés morphologiques des leucocytes, soient maintenues ;
l'identification des classes et, de préférence, également des sous-classes sélectionnées des leucocytes en utilisant une cytométrie en écoulement et /ou une morphologie microscopique.

2. Procédé de la revendication 1, caractérisé en ce que chacun desdits anticorps marqués se compose d'un anticorps lié à un fluorochrome.

3. Procédé de la revendication 2, caractérisé en ce qu'on emploie une combinaison de mesures physiques pour isoler des classes du leucocytes et où la distribution de fluorescence de chaque classe est mesurée.

4. Procédé de la revendication 3, caractérisé en ce qu'au moine l'une desdites mesures physiques est la dispersion de la lumière, l'impédance électronique, une combinaison de dispersion de la lumière sur angle direct et à angle droit ou une combinaison d'impédance électronique et de dispersion de la lumière à angle droit.

5. Procédé de la revendication 3, caractérisé en ce que les leucocytes sont classés par morphologie et sous-classés par intensité de fluorescence.

6. Procédé selon l'une quelconque des revendications 1 à 5, caractérisé en ce que le réactif de fixation contient du glutaraldéhyde.

7. Procédé selon l'une quelconque des revendications 1 à 5, caractérisé en ce que le réactif de fixation contient un dialdéhyde non fluorescent en tant qu'ingrédient actif.

8. Procédé selon l'une quelconque des revendications 1 à 7, carcatérisé en ce que ledit réactif de lyse contient de la saponine à une concentration d'environ 0,24 g par litre jusqu'a environ 4 g par litre ; ou environ 0,2 g par litre à environ 1,14 g par litre, dans l'échantillon lysé.

9. Procédé selon l'une quelconque des revendications 1 à 8, caractérisé en ce que la température de lyse est comprise entre environ 24°C et environ 42°C.

10. Procédé selon l'une quelconque des revendications 1 à 9, caractérisé en ce que la durée de la lyse est de 8 secondes jusqu'à environ 5 minutes.

11. Système réactif a utiliser sur un échantillon de sang entier pour identifier des classes et, de préférence, également des sous-classes sélectionnées de leucocytes, le système réactif comprenant de plus :
des anticorps marqués spécifiques des déterminants antigéniques sur la surface des cellules pour marquer les leucocytes ;
un réactif de lyse pour le traitement de l'échantillon pendant un temps prédéterminé et dans conditions spécifiées de température, contenant une quantité de saponine suffisante pour lyser les érythrocytes, mais laissant les leucocytes sensiblement intacts ; et
un réactif de fixation pour traiter l'échantillon lysé pendant un temps prédéterminé et dans des conditions spécifiées de température, contenant un agent réticulant en une quantité telle que les propriétés physiques et, de préférence, également les propriétés morphologiques des leucocytes soient maintenues pour identifier les classes et, de préférence, également des sous-classes sélectionnées des leucocytes par cytométrie en écoulement et/ou morphologie microscopique.

12. Système réactif de la revendication 11, caractérisé en ce que le réactif de fixation contient du glutaraldéhyde.

13. Système réactif de la revendication 11, caractérisé en ce que ledit réactif de fixation contient un dialdéhyde non fluorescent en tant qu'ingrédient actif.

14. Système réactif selon l'une quelconque des revendications 11 à 13, caractérisé en ce que ledit réactif de lyse contient de la saponine à une concentration d'environ 0,24 g par litre jusqu'à environ 4 g par litre ; ou bien environ 0,2 g par litre à environ 1,14 g par litre dans l'échantillon lysé.

15. Système réactif selon l'une quelconque des revendications 11 à 14, caractérisé par des anticorps liés à un fluorochrome pour marquer les leucocytes.

16. Utilisation d'un système réactif comprenant :
(i) un réactif de lyse contenant de la saponine pour lyser les érythrocytes dans un échantillon de sang entier, tout en laissant les leucocytes sensiblement intacts, des classes et, de préférence, également des sous-classes des leucocytes ayant été marquéesau moyen d'anticorps spécifiques et
(ii) un réactif de fixation contenant un agent réticulant pour maintenir las propriétés physiques et, de préférence, également les propriétés morphologiques des leucocytes dans l'échantillon,
dans la préparation d'un échantillon pour l'identification de classes et, de préférence, également de sous-classes de leucocytes, en utilisant une cytométrie en écoulement et/ou une morphologie microscopique.

17. Utilisation selon la revendication 16, où ledit réactif de fixation contient du glutaraldéhyde.

18. Utilisation selon la revendication 16, où ledit réactif de fixation contient un dialdéhyde non fluorescent en tant qu'ingrédient actif.

19. Utilisation selon l'une quelconque des revendications 16 à 18, où au moins un colorant est utilisé pour teinter les leucocytes.

20. Utilisation selon l'une quelconque des revendications 16 à 19, où ledit réactif de lyse contient de la saponine à une concentration d'environ 0,24 g par litre jusqu'à environ 4 g par litre ou bien environ 0,2 g par litre à environ 1,14 g par litre, dans l'échantillon lysé.

21. Utilisation selon l'une quelconque des revendications 16 à 20, où des anticorps liés à un fluorochrome sont utilisés pour marquer les leucocytes.
